# EUROPÄISCHE PATENTANMELDUNG

(11) **EP 3 275 492 A1**
(43) Veröffentlichungstag der Anmeldung: **31.01.2018**
(21) Anmeldenummer: 16181529.5
(22) Anmeldetag: 27.07.2016
(51) Int. Cl.: A61M 15/00, B65D 83/20, A61M 16/06, B05B 1/00, B05B 11/00, B65D 47/26

(54) **FLÜSSIGKEITSSPENDER, INSBESONDERE INHALATOR**

(71) Anmelder: Aptar Radolfzell GmbH, 78315 Radolfzell (DE)
(72) Erfinder: Greiner-Perth, Jürgen, 78244 Gottmadingen (DE); Schwarz, Jutta, 78333 Stockach (DE)
(74) Vertreter: Patentanwaltskanzlei Cartagena

(57) **Zusammenfassung**

Austragvorrichtungen (30) für einen Flüssigkeitsspender (10) zum Austrag kosmetischer oder pharmazeutischer Flüssigkeiten, insbesondere in Form einer Austragvorrichtung für einen Inhalator, sind aus dem Stand der Technik bekannt.

Es werden gemäß den unterschiedlichen Aspekten der Erfindung Möglichkeiten vorgeschlagen, um eine herstellungstechnisch günstige Montage zu gewährleisten.

Insbesondere wird eine die Austragvorrichtung (30) vorgeschlagen, die über zwei Gehäusebauteil (40, 62) verfügt, die mittels einer Gewindeeinrichtung (80) in einer überlagerten axialen und rotativen Bewegung gegeneinander beweglich sind, wobei die Gewindeeinrichtung (80) an einem ersten der beiden Gehäusebauteite eine helixabschnittsförmige Nut (84b) aufweist.

Die Gewindeeinrichtung (80) weist an einem zweiten der beiden Gehäusebauteite eine Gewindenocke (82) auf, die in die Nut (84B) eingreift. Am ersten Gehäusebauteil (40) ist zusätzlich eine axial erstreckte Einführungsnut (84A) vorgesehen, die an einem Mündungspunkt in die helixabschnittsförmige Nut (84B) mündet.

## Beschreibung

### ANWENDUNGSGEBIET UND STAND DER TECHNIK

Die Erfindung betrifft einen Austragkopf für einen Flüssigkeitsspender nach dem Oberbegriff des Anspruchs 1 sowie nach den Oberbegriffen der Ansprüche 6, 9 und 12. Die Erfindung betrifft weiterhin einen Flüssigkeitsspender mit einer Austragvorrichtung nach dem Oberbegriff des Anspruchs 14.

Aus dem Stand der Technik sind vielfältige Flüssigkeitsspender bekannt. Die Erfindung betrifft insbesondere, jedoch nicht ausschließlich, Flüssigkeitsspender sowie Austragvorrichtungen hierfür, die als mobile Inhalatoren dienen können.

Ein gattungsgemäßer Flüssigkeitsspender, insbesondere in Form eines Inhalators genannter Art, dient dem Zweck, Flüssigkeit in insbesondere vernebelter Form abzugeben, um hierdurch einen pharmazeutischen Zweck zu erfüllen. Umfasst sind jedoch auch baulich ähnliche kosmetische Spender.

Die bekannten Gestaltungen von Inhalatoren gehen üblicherweise mit einer hohen Komplexität und einer Vielzahl von Bauteilen einher, so dass eine Verwendung als Einweg-Produkt problematisch ist.

### AUFGABE UND LÖSUNG

Aufgabe der Erfindung ist es, bauliche Vereinfachungen zur Verfügung zu stellen, die einen bezüglich der Handhabung optimalen Flüssigkeitsspender bei geringen Herstellungskosten ermöglichen. Die Erfindung umfasst mehrere diesbezügliche Aspekte.

Bezüglich eines ersten Aspekts der Erfindung wird eine Austragvorrichtung für einen Flüssigkeitsspender zum Austrag kosmetischer oder pharmazeutischer Flüssigkeiten vorgeschlagen, die folgende Gestaltung aufweist.

Die Austragvorrichtung verfügt über zwei Gehäusebauteile, die mittels einer Gewindeeinrichtung in einer überlagerten axialen und rotativen Bewegung gegeneinander beweglich sind. Diese Gewindeeinrichtung weist an einem ersten der beiden Gehäusebauteile mindestens eine helixabschnittsförmige Nut auf.

Die Gewindeeinrichtung weist an einem zweiten der beiden Gehäusebauteile eine Gewindenocke auf, die in die Nut eingreift. Weiterhin ist am ersten Gehäuseteil eine axial erstreckte Einführungsnut vorgesehen, die an einem Mündungspunkt in die helixabschnittsförmige Nut mündet.

Gemäß diesem Aspekt der Erfindung ist demnach vorgesehen, dass zusätzlich zur gleichsam einen Gewindegang darstellende helixabschnittsförmigen Nut zusätzlich eine axial erstreckte Einführungsnut vorgesehen ist, die in die den Gewindegang darstellende Nut mündet. Dies vereinfacht die Montage der betroffenen Gehäusebauteile erheblich, da zum Zwecke der Montage ein axiales Fügen der beiden Gehäusebauteile ausreicht. Die Gewindenocke kann daher an einer Stirnseite des mit der Nut versehenen Gehäusebauteils zunächst in die axial erstreckte Einführungsnut eingeschoben werden und gelangt so bis zur gewindetypischen helixabschnittsförmigen Nut.

Obwohl im Rahmen dieser Beschreibung meist auf eine helixabschnittsförmige Nut sowie eine Einführungsnut Bezug genommen wird, können diese auch mehrfach vorgesehen sein. So können insbesondere zwei oder drei oder auch mehr helixabschnittsförmige Nuten, gegebenenfalls mit jeweils einer Einführungsnut, vorgesehen sein, um eine besonders saubere Führung der Gehäusebauteile gegeneinander zu gewährleisten.

Dies kann zum einen genutzt werden, um die beiden Gehäusebauteile in einer Art fest miteinander zu verbinden, wie es üblicherweise mit Gewindeeinrichtungen geschieht. Dies bedeutet, dass die beiden Gehäusebauteile im Lieferzustand des Austragkopfes als Teil eines Flüssigkeitsspenders in einer festen Relativlage angeordnet sind, die bestimmungsgemäß im Betrieb keine Änderung mehr erfährt. Bei einer solchen Gestaltung weist die helixabschnittsförmige Nut eine Formgebung auf, die gemeinsam mit der Gewindenocke zu einer Selbsthemmung im Zuge des auf das axiale Fügen folgenden Festdrehens führt.

Bevorzugt ist jedoch eine Gestaltung, bei der die beiden Gehäusebauteile im Betrieb des Spenders bestimmungsgemäß durch Relativverlagerung zueinander einen Betriebsparameter des Spenders ändern, insbesondere das Öffnen und Schließen eines Auslassventils bewirken.

In einem solchen Fall ist somit vorgesehen, dass die beiden Gehäusebauteile, die vorzugsweise einer Basis und einem Austragkopf des Spenders zugeordnet sind, durch eine überlagerte axiale und rotative Relativbewegung zueinander geeignet sind, insbesondere den Austrag von Flüssigkeit mittels des Spenders auszulösen und zu beenden. Eine solche Relativbeweglichkeit zur Steuerung des Austrags hat sich als bei der Benutzung sehr intuitiv und gut dosierbar herausgestellt. Insbesondere kann der Benutzer ein Atemstück am Austragkopf durch Umschließen mit den Lippen oder dergleichen fixieren und durch Drehen des mit der Basis drehfest verbundenen Flüssigkeitsspeichers den Austrag beginnen und beenden.

Der Mündungspunkt, an dem die axial erstreckte Nut in die helixabschnittsförmige Nut mündet, ist vorzugsweise an einem der Enden der helixabschnittsförmigen Nut vorgesehen. Wenn die Gewindenocke bei der Einführung in die Einführnut diesen Mündungspunkt erreicht, ist eine Relativbeweglichkeit innerhalb der helixabschnittsförmigen Nut nur noch in eine Richtung gegeben.

Vorzugsweise ist die Gewindenocke auch im Lieferzustand des Spenders am Mündungspunkt, also beim Übergang von der axial erstreckten Nut in die helixabschnittsförmige Nut, angeordnet. Um hier die Relativlage zu sichern, kann innerhalb der Nut eine Widerstandsgeometrie vorgesehen sein, im Bereich derer die Bewegung der Gewindenocke eines höheren Kraftaufwandes bedarf. Für den Benutzer wird somit das Verlassen dieser Endlage der Gewindenocke in der helixabschnittsförmigen Nut haptisch erfassbar. Hiermit verwandt ist auch der zweite Aspekt der vorliegenden Erfindung.

Bezüglich dieses zweiten Aspekts der Erfindung wird eine Austragvorrichtung vorgeschlagen, die folgende Gestaltung aufweist, wobei die Gestaltung vorzugsweise auch die Hauptmerkmale der vorbeschriebenen Gestaltung aufweist.

Die Austragvorrichtung gemäß diesem zweiten Aspekt verfügt über zwei Gehäusebauteile, die mittels einer Gewindeeinrichtung in einer überlagerten axialen und rotativen Bewegung gegeneinander beweglich sind. Diese Gewindeeinrichtung weist an einem ersten der beiden Gehäusebauteile mindestens eine helixabschnittsförmige Nut auf.

In der Nut ist ein mit der Gewindenocke zusammenwirkender Blockierabschnitt vorgesehen, wobei ein Überdrücken der Gewindenocke über den Blockierabschnitt in einer Fügerichtung möglich ist und in einer Trennrichtung verhindert wird.

Der in der Nut angeordnete Blockierabschnitt sorgt dafür, dass die Relativbeweglichkeit der Gehäusebauteile zueinander bei Erreichen des Blockierabschnitts durch die Gewindenocke nur in eine Richtung möglich ist. Somit ist eine Trennung der zwei Gehäusebauteile nach initialem Einführen der Gewindenocke in die Nut nicht mehr möglich. Von besonderem Vorteil ist dies im Zusammenhang mit der bereits erläuterten axial erstreckten Einführungsnut. Es gestattet, alleine durch eine axiale Bewegung der beiden Gehäusebauteile diese untrennbar miteinanderzu fügen.

Der Blockierabschnitt und/oder die Gewindenocke ist/sind vorzugsweise mit einer Rampe versehen, die derart ausgerichtet sind, dass sie vor dem Fügen in Richtung des jeweils anderen Elements weist und beim Fügen der Gehäusebauteile diese Rampe die Vorbeiführung der Nocke am Blockierabschnitt erleichtert.

Weiterhin ist/sind der Blockierabschnitt und/oder die Gewindenocke vorzugsweise mit einer Anschlagsfläche versehen, die derart ausgerichtet sind, dass sie nach dem Fügen in Richtung des jeweils anderen Elements weist und nach dem Fügen eine Trennung der Gehäusebauteile verhindert.

Somit wird die Vorbeiführung der Gewindenocke am Blockierabschnitt dadurch ermöglicht, dass der Blockierabschnitt und/oder die Gewindenocke vorzugsweise eine bezogen auf die Fügerichtung asymmetrische Formgebung aufweisen. So kann beispielsweise durch Rampen beidseitig am Blockierabschnitt und an der Gewindenocke erreicht werden, dass in Fügerichtung nur eine geringe zusätzliche Kraft erforderlich ist, um die Gewindenocke über den Blockierabschnitt zu drücken, während in entgegengesetzter Richtung die Anschlagsflächen, die vorzugsweise orthogonal zur Fügerichtung/Trennrichtung ausgerichtet sind, in Anlage aneinander kommen und eine Trennung deutlich erschweren oder sogar eine zerstörungsfreie Trennung der beiden Gehäusebauteile verhindern.

Ein besonderer Vorteil ergibt sich, wenn der Blockierabschnitt am Ende der Einführungsnut vorgesehen ist und eine in Fügerichtung und somit auf den Mündungspunkt der axial erstreckten Einführungsnut hinweisende Anschlagsfläche aufweist. Diese ist dann in etwa fluchtend zu einer seitlichen Begrenzungsfläche der helixabschnittsförmigen Nut anzuordnen, so dass der Blockierabschnitt gleichsam im Betrieb mit seiner Anschlagsfläche einen Teil der seitlichen Wandung der helixabschnittsförmigen Nut bildet.

Bezüglich eines dritten Aspekts der Erfindung wird eine Austragvorrichtung vorgeschlagen, die folgende Gestaltung aufweist, wobei die Gestaltung vorzugsweise auch die Hauptmerkmale einer der vorbeschriebenen Gestaltungen aufweist.

Die Austragvorrichtung gemäß diesem dritten Aspekt umfasst eine Basis und einen Austragkopf, der zur Betätigung eines Auslassventils gegenüber der Basis in einer überlagerten axialen und rotativen Bewegung verlagerbar ist, wobei die überlagerte Relativbeweglichkeit durch eine Gewindeeinrichtung bewirkt wird, die zwischen der Basis und dem Austragkopf angeordnet ist.

Die Basis weist ein erstes Bauteil mit einem hülsenförmigen Führungsabschnitt auf und der Austragkopf weist ein zweites Bauteil mit einem hülsenförmigen Führungsabschnitt auf. Zwischen einer Außenfläche des Führungsabschnitts der Basis und einer Innenfläche des Führungsabschnitts des Austragkopfes ist die Gewindeeinrichtung vorgesehen. Der Austragkopf verfügt über ein Zuströmrohr, welches in den Führungsabschnitts der Basis hineinragt und dort mittels einer an der Basis vorgesehenen Gleithülse zentriert ist.

Die hülsenförmigen Führungsabschnitte an der Basis zum einen und am Austragkopf zum anderen gewährleisten eine stabile Führung und bilden gemeinsam eine Gewindeeinrichtung, die eine überlagerte axiale und rotative Beweglichkeit zwischen dem Austragkopf und der Basis definiert. Um im Bereich dieser Führungsflächen ein Verkanten oder ähnliche Störungen zu vermeiden, sind die hülsenförmigen Führungsabschnitte vorzugsweise mit einem relativ großen Durchmesser versehen. Dies führt jedoch dazu, dass das Zuführrohr am Austragkopf vergleichsweise ungenau zentriert ist, was bei der Montage zu Schwierigkeiten führen kann.

Durch die zusätzliche Gleithülse an der Basis, innerhalb derer das Zuführrohr des Austragkopfes schraubenförmig beweglich ist, kann dennoch gewährleistet werden, dass dieses Zuführrohr stets zentriert gehalten wird. Es hat sich herausgestellt, dass die Störungen in der Produktion beim Aufsetzen des Austragkopfes auf einen Flüssigkeitsspeicher durch diese zusätzliche Zentrierung deutlich verringert werden.

Da das Zuführrohr innerhalb der Gleithülse sowohl axial als auch rotativ beweglich sein muss, ist es von Vorteil, wenn das Zuführrohr eine kreisrunde Außenform aufweist und die Gleithülse eine entsprechende kreisrunde Innenform aufweist. Die Gleithülse ist vorzugsweise als separate konzentrische Hülse zum hülsenförmigen Führungsabschnitt der Basis ausgebildet und mit diesem fest, insbesondere einstückig, verbunden. Der entsprechende radial erstreckte Verbindungsabschnitt liegt vorzugsweise in Form mehrerer speichenartiger Verbindungsabschnitte vor, die den basisseitigen Führungsabschnitt mit der Gleithülse verbinden.

Die Gleithülse ist vorzugsweise derart an der Basis und dort am hülsenförmigen Führungsabschnitt vorgesehen, dass das Zuströmrohr selbst in jener Endlage, in der es maximal weit aus der Gleithülse herausgezogen ist, immer noch durch diese hindurchragt. Insbesondere in dieser Relativstellung erfolgt vorzugsweise die Montage, so dass es erheblich ist, dass das Zuströmrohr sich bis unter das untere Ende der Gleithülse erstreckt.

Bezüglich eines vierten Aspekts der Erfindung wird eine Austragvorrichtung vorgeschlagen, die folgende Gestaltung aufweist, wobei die Gestaltung vorzugsweise auch die Merkmale einer der vorbeschriebenen Gestaltungen aufweist.

Die Austragvorrichtung gemäß diesem vierten Aspekt der Erfindung verfügt über einen Austragkopf mit einer Austragöffnung, durch die hindurch die Flüssigkeit in eine umgebende Atmosphäre abgegeben werden kann. Der Austragkopf weist ein äußeres Gehäusebauteil und mindestens zwei innere Gehäusebauteile auf, wobei die beiden inneren Gehäusebauteile gemeinsam flüssigkeitsführende Abschnitte des Austragkopfes begrenzen, mittels derer Flüssigkeit zur Austragöffnung geleitet wird.

Die beiden inneren Gehäusebauteile sind zur isolierten Abdichtung der flüssigkeitsführenden Abschnitte in einer ersten Montagerichtung aneinander gedrückt. Gemeinsam sind die beiden inneren Gehäusebauteile in einer von der ersten Montagerichtung abweichenden zweiten Montagerichtung in das Außengehäuse eingeschoben, so dass das Außengehäuse eine Trennung der beiden inneren Gehäusebauteile unterbindet.

Die beschriebene Bauform stellt eine vorteilhafte Möglichkeit dar, wenn am Austragkopf mehrere innere Gehäusebauteile vorgesehen sind, die gemeinsam flüssigkeitsführende Bereiche begrenzen. Eine solche Gestaltung kann insbesondere geboten sein, wenn zusätzliche Bauteile in den durch die beiden inneren Gehäusebauteile begrenzten Fluidbereich eingesetzt werden sollen, da eine einstückige Gestaltung der inneren Gehäusebauteile dann nicht möglich ist. Zu diesen zusätzlichen Teilen können beispielsweise Filter sowie Austrittsdüsen, insbesondere in Form von Düsenplatten, gehören. Bei

einer Gestaltung gemäß diesem Aspekt der Erfindung sind die beiden inneren Bauteile getrennte Bauteile, die ineinander eingeschoben oder aufeinander aufgeschoben werden. Eine konstruktiv mitunter schwierige sichere Fügung dieser Bauteile kann dadurch entfallen, dass der Verbund der beiden inneren Gehäusebauteile in das genannte äußere Gehäusebauteil eingeschoben wird und somit das äußere Gehäusebauteil die inneren Gehäusebauteile gleichsam in Art einer Klammer gegen Trennung sichert.

Eines der inneren Gehäusebauteile weist vorzugsweise einen Auslasskanalabschnitt auf, der nach Einschub der inneren Gehäusebauteile in das äußere der Gehäusebauteile fluchtend zu einer Durchbrechung des Außengehäuses angeordnet ist.

Weiterhin sind das Außengehäuse und mindestens eines der inneren Gehäusebauteile vorzugsweise derart aufeinander abgestimmt, dass es zu einem Einrasten des Verbundes der beiden inneren Gehäusebauteile kommt, wenn diese in das äußere Gehäusebauteil eingeschoben werden.

Weiterhin kann es insbesondere von Vorteil sein, wenn auf der Innenseite des Außengehäuses oder an der Außenseite eines oder beider innerer Gehäusebauteile Einführschrägen vorgesehen sind, die das Einführen des Verbundes aus den beiden inneren Gehäusebauteilen erleichtern und dafür sorgen, dass die inneren Gehäusebauteile im eingeschobenen Zustand fluiddicht aneinander angepresst werden.

Die Erfindung begrifft darüber hinaus auch einen Flüssigkeitsspender zum Austrag kosmetischer oder pharmazeutischer Flüssigkeiten, insbesondere in Form eines Inhalator, der über einen Flüssigkeitsspeicher und über eine Austragvorrichtung zum Austrag von Flüssigkeit aus dem Flüssigkeitsspeicher gemäß einer der oben beschriebenen Gestaltungen verfügt.

Der Flüssigkeitsspender ist vorzugsweise als Druckspeicher ausgebildet und ist weiterhin vorzugsweise zur Aufnahme von maximal 500 ml ausgelegt, vorzugsweise zur Aufnahme von maximal 250 ml ausgebildet.

Der Flüssigkeitsspeicher ist vorzugsweise mit einer der folgenden Flüssigkeiten befüllt, die insbesondere vorzugsweise in Form eines Sprühnebels zum Zwecke der Inhalation ausgetragen werden: eine salzhaltige wässrige Lösung oder eine wässrige Lösung in Form einer Ringerlösung oder einer gepufferten Lösung oder eine wässrige Lösung mit mindestens einem der Zusätze Kohlenhydrate, ätherische Öle, Menthol und Pflanzenextrakte oder eine wässrige Lösung, enthaltend Vitamine, Spurenelemente, Mangan oder Zink, oder eine wässrige Lösung mit mindestens einem der Zusätze aus der Gruppe umfassend Zimtöl, Teebaumöl, Salbeiöl, Thymianöl, Zitronenmelissenöl.

Der Flüssigkeitsspender ist vorzugsweise als Inhalator ausgebildet und verfügt über ein Atemstück, welches als Mundstück oder als Atemmaske ausgestaltet ist.

### KURZBESCHREIBUNG DER ZEICHNUNGEN

Weitere Vorteile und Aspekte der Erfindung ergeben sich aus den Ansprüchen und aus der nachfolgenden Beschreibung eines bevorzugten Ausführungsbeispiels der Erfindung, das nachfolgend anhand der Figuren erläutert ist.
Fig. 1 und 2 zeigen einen erfindungsgemäßen Flüssigkeitsspender in einer Gesamtdarstellung sowie einer geschnittenen Darstellung.
Fig. 3A und 3B verdeutlichen die Relativbeweglichkeit des Austragkopfes und der Basis des Austragkopfes der Fig. 1 und 2 zum Zwecke des Öffnens+ und Schließens des Auslassventils.
Fig. 4 und 5 zeigen die für diese Relativbeweglichkeit verantwortliche Gewindeeinrichtung.
Fig. 6 zeigt die Wirkweise der Gleithülse an der Basis des erfindungsgemäßen Spenders.
Fig. 7A bis 7C zeigen die Montage des Austragkopfes des Spenders.

### DETAILLIERTE BESCHREIBUNG DESAUSFÜHRUNGSBEISPIELS

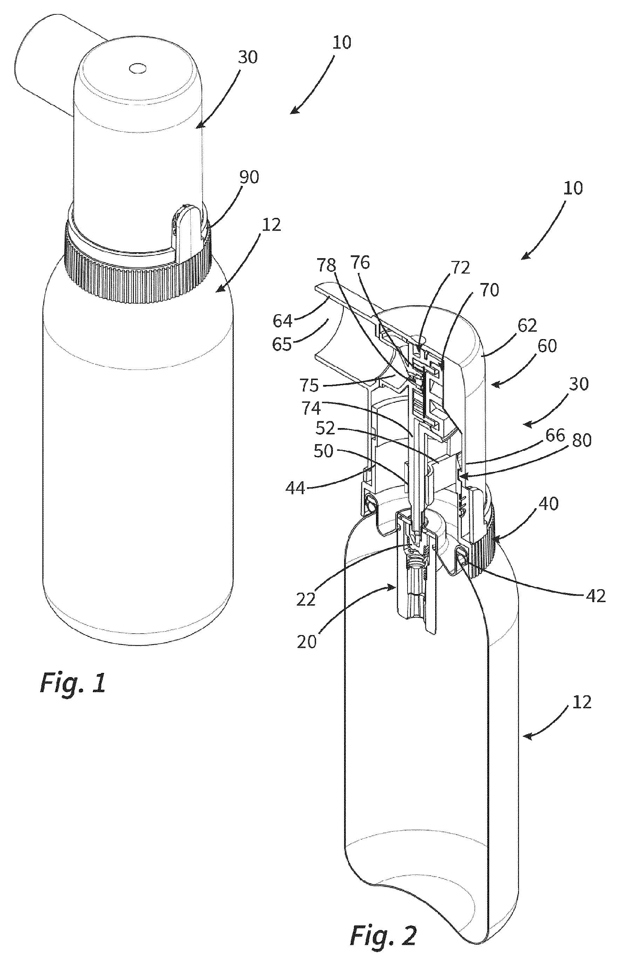

Die Fig. 1 und 2 zeigen einen erfindungsgemäßen Flüssigkeitsspender 10, der vorliegend als Inhalator ausgebildet ist, in einer Gesamtdarstellung sowie in einer geschnittenen Darstellung.

Der Flüssigkeitsspender 10 verfügt über eine Austragvorrichtung 30, die auf einen als Druckspeicher ausgebildeten Flüssigkeitsspeicher 12 aufgesetzt ist. Zwischen dem Flüssigkeitsspeicher 12 und der Austragvorrichtung 30 ist eine Ventileinrichtung 20 vorgesehen, die über einen durch axiales Niederdrücken verlagerbaren Ventilkörper 22 geöffnet werden kann und nach Wegfall der axialen Beaufschlagung durch eine Ventilfeder zurück in die geschlossene Ausgangslage gedrückt wird.

Die Austragvorrichtung 30 verfügt über eine Basis 40, die mittels einer Klemmgeometrie 42 drehfest am Flüssigkeitsspeicher 12 befestigt ist. Diese Basis 40 stellt einen hülsenförmigen Führungsabschnitt 44 zur Verfügung, an der ein Austragkopf 60 der Austragvorrichtung 30 beweglich geführt ist.

Der Austragkopf 60 weist ein Außengehäuse-Bauteil 62 auf, innerhalb dessen zwei Innenbauteile 70, 72 angeordnet sind, die ortsfest zum Außengehäuse-Bauteil 62 befestigt sind und gemeinsam einen Flüssigkeitspfad von der Ventileinrichtung 20 durch ein Zuströmrohr 74 bis zu einem Flüssigkeitsauslass 75 begrenzen. In dem durch die beiden Innenbauteile 70, 72 gemeinsam definierten Innenraum sind eine Düseneinheit 78 mit einer nicht näher dargestellten Düsenplatte sowie eine Filtereinheit 76 mit Membran angeordnet. Das Zuströmrohr 74 ragt bis zum Ventilkörper 22 und ist in der Lage, diesen zum Öffnen der Ventileinrichtung kraftzubeaufschlagen.

Nach Entfernen eines Originalitätsabschnittes 90 ist der Austragkopf 60 gegenüber der Basis 40 beweglich. DerAustragkopf 60 kann dann in einer überlagerten rotativen und axialen Bewegung geführt gegenüber der Basis 40 verlagert werden, um hierdurch mittels des Zuströmrohrs 74 die Ventileinrichtung 20 zu öffnen und zu schließen.

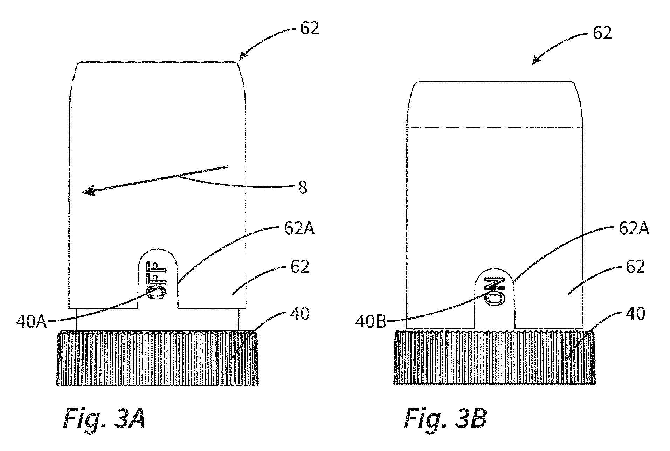

In Fig. 3A ist der Zustand des Flüssigkeitsspenders 10 mit geschlossener Ventileinrichtung 20 dargestellt. In diesem Zustand ist der Austragkopf 60 maximal gegenüber der Basis 40 beabstandet, so dass das Zuströmrohr 74 in seiner oberen Endlage angeordnet ist und daher den Ventilkörper 22 nicht aus seiner Schließlage nach unten drückt. Dieser geschlossene Zustand wird in einem Sichtfenster 62A mittels der Beschriftung "OFF" gekennzeichnet.

Ausgehend hiervon kann der Austragkopf 60 in Richtung des Pfeils 8 gegenüber der Basis 40 gleichzeitig verdreht und axial nach unten verlagert werden. Diese axiale Verlagerung bewirkt, dass das in Fig. 2 dargestellte Zuströmrohr 74 als Teil des Austragkopfes 60 nach unten verlagert wird und dadurch die Ventileinrichtung 20 öffnet. In der Praxis erfolgt diese Relativbewegung jedoch durch Bewegung des Flüssigkeitsspeichers mitsamt der Basis 40 gegenüber dem Austragkopf 60, dessen Atemstück 64 ein Benutzer mit den Lippen umschließt oder dessen Atemstück 64 mittels einer daran angesetzten Atemmaske, die an das Gesicht angedrückt wird, fixiert ist.

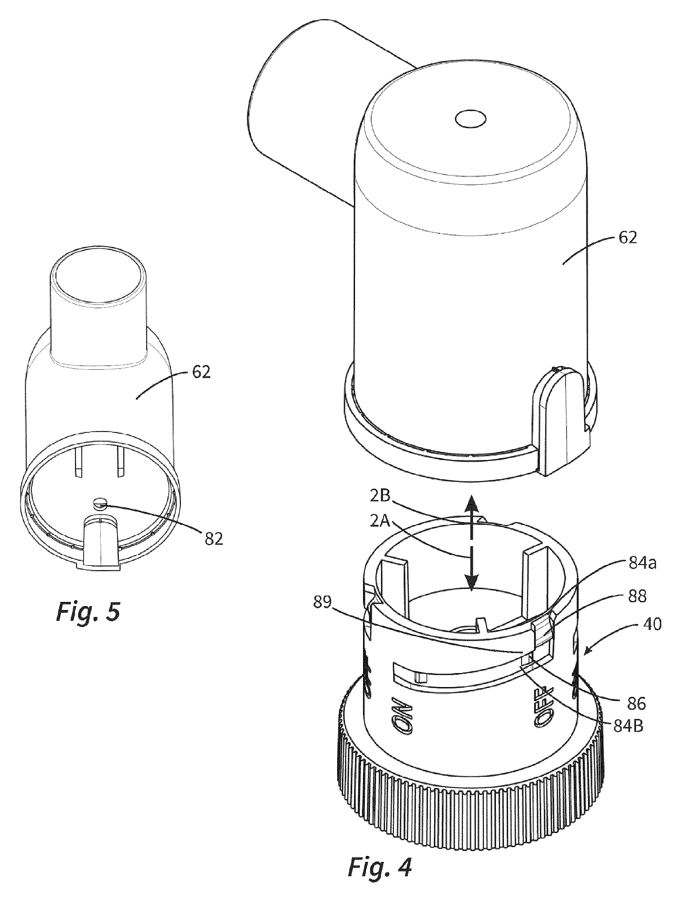

Die bauliche Gestaltung zur Erzielung der überlagerten Beweglichkeit sowie Besonderheiten in diesem Kontext werden anhand der Fig. 4 und 5 verdeutlicht.

Aus Fig. 4 ist ersichtlich, dass das Basisteil über eine helixabschnittsförmige Gewindenut 84B verfügt. Korrespondierend hierzu ist an der Innenseite des Außengehäuse-Bauteils 62 eine Gewindenocke 82 vorgesehen, die im Betrieb entlang der Gewindenut 84B verlagerbar ist, um in den Endlagen einen der Zustände der Fig. 3A und 3B einzunehmen.

Zum Zwecke einer einfachen Montage ist an der Basis 40 zusätzlich eine axial erstreckte Nut 84A vorgesehen, die bezogen auf Fig. 4 am rechten Ende in die helixabschnittsförmige Nut 84B mündet. Innerhalb dieser axial erstreckten Nut 84A ist ein Blockierabschnitt 88 vorgesehen, der nach oben weisend eine Einführschräge aufweist und nach unten weisend über eine Anschlagsfläche verfügt, die mit der oberen Begrenzungsfläche 89 der helixabschnittsförmigen Nut 84B fluchtet. Diese Nut 84A ermöglicht es, ausgehend von der Relativlage des Außengehäuse-Bauteils 62 zur Basis 40 der Fig. 4 im Zuge der Montage lediglich das Außengehäuse-Bauteil 62 in Montagerichtung 2A auf die Basis 40 aufzuschnappen, wobei hierdurch die Gewindenocke 82 bis in die helixabschnittsförmige Nut 84B gelangt. Sobald die Gwindenocke 82 über den Blockierabschnitt 88 hinübergedrückt ist, sind das Außengehäuse-Bauteil 62 und die Basis 40 sicher miteinander verbunden.

Die Gewindeeinrichtung 80 kann somit ohne rotative Bewegung im Zuge der Montage gefügt werden. Da es vorliegend gewünscht ist, dass die Gewindenocke 82 vor Inbetriebnahme nicht entlang der helixabschnittsförmigen Nut 84B versehentlich verlagert wird und zusätzlich der deaktivierte Zustand des Spenders später durch den Nutzer haptisch erfassbar sicherbar sein soll, ist zusätzlich eine Widerstandsgeometrie 86 in der Nut 84B vorgesehen, die ein Passieren der Gewindenocke 82 nur unter erhöhtem Kraftaufwand gestattet.

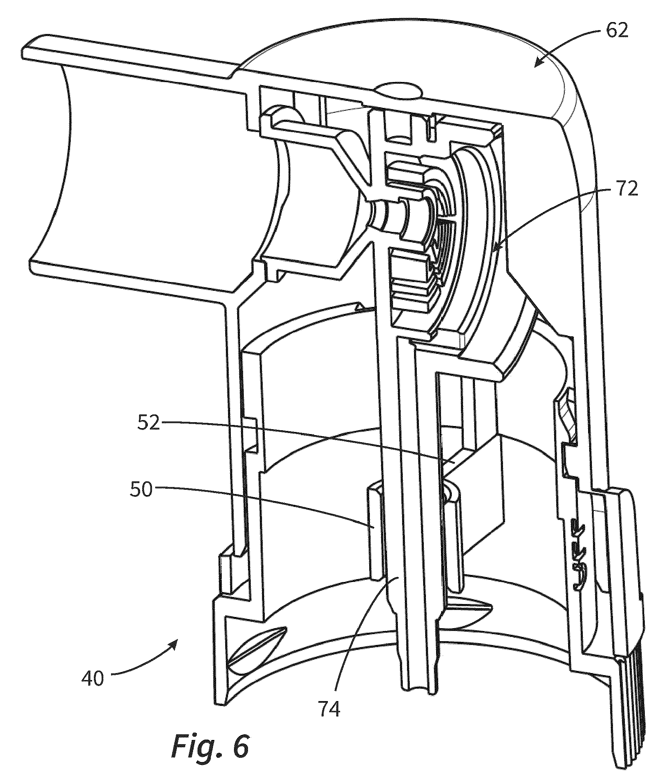

Fig. 6 verdeutlicht, wie der Austragkopf 60 an der Basis 40 geführt ist. Diese Führung erfolgt zum einen an den hülsenförmigen Abschnitten 44, 66 der Basis 40 und des Außengehäuse-Bauteils 62. Zusätzlich ist jedoch eine Gleithülse 50 vorgesehen, die über drei um jeweils 120° beabstandete speichenartige Verbindungsabschnitte 52 zentrisch im hülsenförmigen Abschnitt 44 der Basis 40 gehalten wird. Durch diese Gleithülse 50 hindurch ragt das Zuführrohr 74. Die Austragvorrichtung der Fig. 6 kann somit bei hoher Verfahrenssicherheit auf den Flüssigkeitsspeicher 12 und die Ventileinrichtung 20 aufgesetzt werden. Wäre die Gleithülse 50 nicht vorhanden, so wäre hier ein Blockieren aufgrund mangelnder Zentrierung zu befürchten.

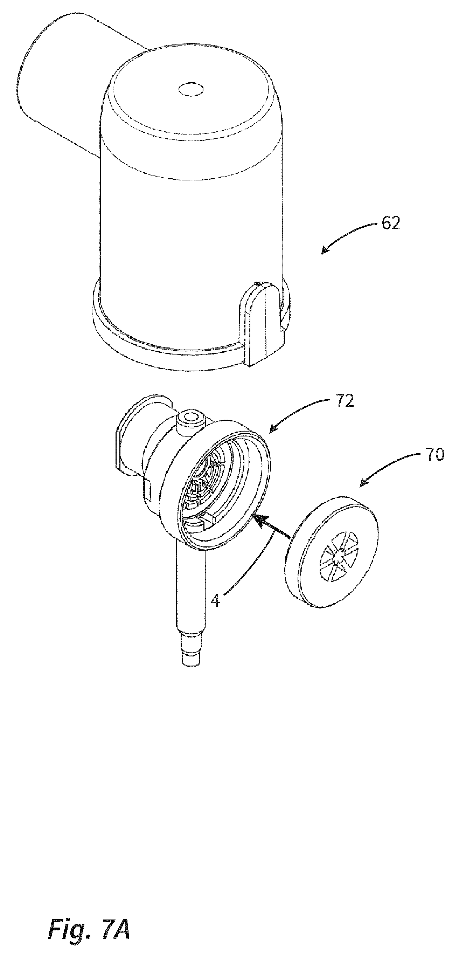

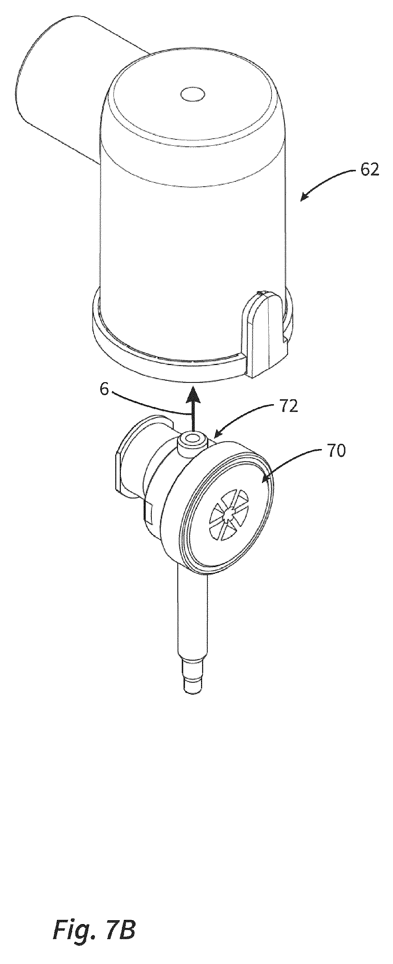

Die Montage der genannten Innenbauteile 70, 72 im Außengehäuse-Bauteil 62 wird anhand der Fig. 7A bis 7C verdeutlicht. Ausgehend von den drei genannten Einzelteilen wird zunächst das Innenbauteil 70, welches eine Art Stopfen darstellt, in einer ersten Montagerichtung 4 in das zweite Innenbauteil 72 eingeschoben, in welchem zuvor in nicht dargestellter Weise die Düseneinheit 78 und die Filtereinheit 76 eingesetzt wurden.

Anschließend wird der Verbund aus den beiden inneren Gehäusebauteilen 70, 72 gemeinsam in einer zweiten Montagerichtung 6 in das Außengehäuse-Bauteil 62 eingeschoben.

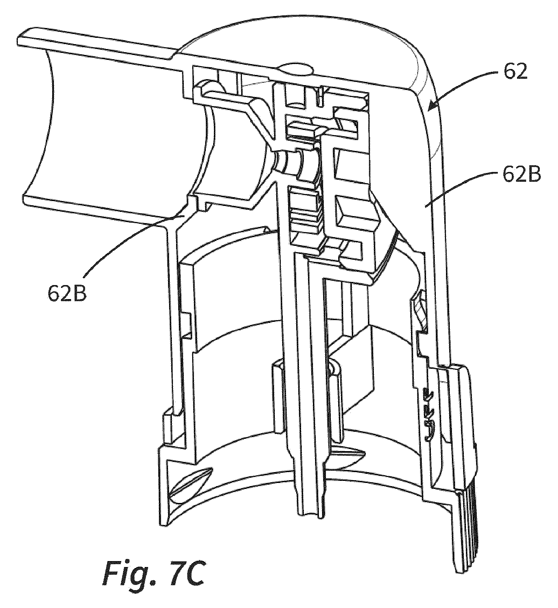

Wie Fig. 7C zeigt, sind die beiden Innenbauteile 70, 72 hierdurch im montierten Zustand im Außengehäuse-Bauteil 62 gleichsam eingeklemmt und hierdurch sicher gegen Trennen gesichert.

## Patentansprüche

1. Austragvorrichtung (30) für einen Flüssigkeitsspender (10) zum Austrag kosmetischer oder pharmazeutischer Flüssigkeiten, insbesondere in Form einer Austragvorrichtung für einen Inhalator, mit den folgenden Merkmalen:
a. die Austragvorrichtung (30) verfügt über zwei Gehäusebauteil (40, 62), die mittels einer Gewindeeinrichtung (80) in einer überlagerten axialen und rotativen Bewegung gegeneinander beweglich sind,
b. die Gewindeeinrichtung (80) weist an einem ersten der beiden Gehäusebauteile mindestens eine helixabschnittsförmige Nut (84B) auf,
**gekennzeichnet durch** die Merkmale
c. die Gewindeeinrichtung (80) weist an einem zweiten der beiden Gehäusebauteile eine Gewindenocke (82) auf, die in die Nut (84B) eingreift,
d. am ersten Gehäusebauteil (40) ist eine axial erstreckte Einführungsnut (84A) vorgesehen, die an einem Mündungspunkt in die helixabschnittsförmige Nut (84B) mündet.

2. Austragvorrichtung (30) nach Anspruch 1 mit dem folgenden Merkmal:
a. das dem Mündungspunkt abgewandten Ende der Einführungsnut (84A) ist an einer Stirnseite des ersten Gehäusebauteils (40) vorgesehen.

3. Austragvorrichtung nach Anspruch 1 oder 2 mit dem folgenden Merkmal:
a. die beiden Gehäusebauteile sind mittels der Gewindeeinrichtung in einer definierten Relativlage zueinander fixiert und werden bestimmungsgemäß im Betrieb des Spenders nicht gegeneinander verlagert.

4. Austragvorrichtung (30) nach Anspruch 1 oder 2 mit dem folgenden Merkmal:
a. die beiden Gehäusebauteile (40, 62) sind dafür ausgebildet, im Betrieb des Flüssigkeitsspenders (10) bestimmungsgemäß durch Relativverlagerung gegeneinander einen Betriebsparameter des Spenders zu verändern, insbesondere das Öffnen und Schließen einer Ventileinrichtung (20) zu bewirken.

5. Austragvorrichtung (30) nach einem der vorsehenden Ansprüche mit dem folgenden Merkmal:
a. der Mündungspunkt ist an einem Ende der helixabschnittsförmigen Nut (84B) vorgesehen und
b. in der helixabschnittsförmigen Nut (84B) ist mindestens eine Widerstandsgeometrie (86) vorgesehen, im Bereich derer die Bewegung der Gewindenocke (82) eines höheren Kraftaufwandes bedarf, wobei die Widerstandsgeometrie (86) vorzugsweise benachbart zum Mündungspunkt angeordnet ist.

6. Austragvorrichtung (30) für einen Flüssigkeitsspender (10) zum Austrag kosmetischer oder pharmazeutischer Flüssigkeiten, insbesondere in Form einer Austragvorrichtung für einen Inhalator, mit den folgenden Merkmalen:
a. die Austragvorrichtung (30) verfügt über zwei Gehäusebauteile (40, 62), die mittels einer Gewindeeinrichtung (80) in einer überlagerten axialen und rotativen Bewegung gegeneinander beweglich sind, und
b. die Gewindeeinrichtung (80) weist an einem ersten der beiden Gehäusebauteile mindestens eine zumindest abschnittsweise helixförmige Nut (84A, 84B) auf,
**gekennzeichnet durch** die Merkmale:
c. die Gewindeeinrichtung (80) weist an einem zweiten der beiden Gehäusebauteile eine Gewindenocke (82) auf, die in die Nut (84B) eingreift,
d. in der Nut (84A) ist ein mit der Gewindenocke (82) zusammenwirkender Blockierabschnitt (88) vorgesehen, wobei ein Überdrücken der Gewindenocke (82) über den Blockierabschnitt (88) in einer Fügerichtung (2A) möglich ist und in einer Trennrichtung (2B) verhindert wird,
wobei die Austragvorrichtung (30) vorzugsweise zusätzlich die Merkmale eines der vorstehenden Ansprüche aufweist.

7. Austragvorrichtung (30) nach Anspruch 6 mit mindestens einem der folgenden Merkmalen:
a. der Blockierabschnitt (88) und/oder die Gewindenocke (82) ist mit einer Rampe versehen, die derart ausgerichtet sind, dass sie vor dem Fügen in Richtung des jeweils anderen Elements weist und beim Fügen der Gehäusebauteile diese Rampe die Vorbeiführung der Nocke am Blockierabschnitt erleichtert, und/oder
b. der Blockierabschnitt (88) und/oder die Gewindenocke (82) ist mit einer Anschlagsfläche versehen, die derart ausgerichtet sind, dass sie nach dem Fügen in Richtung des jeweils anderen Elements weist und nach dem Fügen eine Trennung der Gehäusebauteile verhindert.

8. Austragvorrichtung (30) nach Anspruch 6 oder 7 mit den folgenden Merkmalen:
a. der Blockierabschnitt (88) weist eine in Fügerichtung weisende Anschlagsfläche auf, und
b. die Anschlagsfläche ist am Mündungspunkt der axialen Nut derart angeordnet, dass sie fluchtend zu einer seitlichen Begrenzungsfläche (89) der helixabschnittsförmigen Nut (84B) die Nut begrenzt.

9. Austragvorrichtung (30) für einen Flüssigkeitsspender (10) zum Austrag kosmetischer oder pharmazeutischer Flüssigkeiten, insbesondere in Form einer Austragvorrichtung für einen Inhalator, mit den folgenden Merkmalen:
a. die Austragvorrichtung (30) umfasst eine Basis (40) und
b. die Austragvorrichtung umfasst einen Austragkopf (60), der zur Betätigung einer Ventileinrichtung (20) gegenüber der Basis (40) in einer überlagerten axialen und rotativen Bewegungverlagerbar ist, und
c. die überlagerte Relativbeweglichkeit wird durch eine Gewindeeinrichtung (80) bewirkt, die zwischen der Basis (40) und dem Austragkopf (60) angeordnet ist,
**gekennzeichnet durch** die Merkmale:
e. die Basis (40) weist ein erstes Bauteil mit einem hülsenförmigen Führungsabschnitt (44) auf,
f. der Austragkopf (60) weist ein zweites Bauteil (62) mit einem hülsenförmigen Führungsabschnitt (66) auf,
g. die Gewindeeinrichtung (80) ist zwischen einer Außenfläche des Führungsabschnitts (44) der Basis (40) und einer Innenfläche des Führungsabschnitts (66) des Austragkopfes (60) vorgesehen,
h. der Austragkopf (60) verfügt über ein Zuströmrohr (74), welches in den Führungsabschnitts (44) der Basis (40) hineinragt und dort mittels einer an der Basis (40) vorgesehenen Gleithülse (50) zentriert ist,
wobei die Austragvorrichtung (30) vorzugsweise zusätzlich die Merkmale eines der vorstehenden Ansprüche aufweist.

10. Austragvorrichtung (30) nach Anspruch 9 mit dem Merkmal:
a. die Gleithülse (50) ist als vom hülsenförmigen Führungsabschnitt (44) der Basis getrennte Gleithülse (50) ausgebildet und mittels mindestens eines Verbindungsabschnitts (52), insbesondere mittels mehrerer speichenartiger Verbindungsabschnitte (52), mit dem Führungsabschnitt (44) der Basis (40) verbunden.

11. Austragvorrichtung (30) nach einem der Ansprüche 9 oder 10 mit den Merkmalen:
a. der hülsenförmige Führungsabschnitt (44) der Basis (40) und der hülsenförmige Führungsabschnitt (66) des Austragkopfes (60) sind gegeneinander zwischen zwei Endlagen axial relativbeweglich, und
b. die Gleithülse (50) und das Zuströmrohr (74) sind derart aufeinander abgestimmt, dass das Zuströmrohr in beiden Endlagen durch die Gleithülse (50) hindurchragt.

12. Austragvorrichtung (30) für einen Flüssigkeitsspender (10) zum Austrag kosmetischer oder pharmazeutischer Flüssigkeiten, insbesondere in Form einer Austragvorrichtung für einen Inhalator, mit den folgenden Merkmalen:
a. die Austragvorrichtung (30) verfügt über einen Austragkopf (60) und
b. der Austragkopf (60) weist eine Austragöffnung (65) auf, durch die hindurch die Flüssigkeit in eine umgebende Atmosphäre abgegeben werden kann, und
c. der Austragkopf (60) weist ein äußeres Gehäuse-Bauteil (62) und mindestens zwei innere Gehäusebauteile (70,72) auf, und
d. die beiden inneren Gehäusebauteile (70, 72) begrenzen gemeinsam flüssigkeitsführende Abschnitte des Austragkopfes (60), mittels derer FlüssigkeitzurAustragöffnung (65) geleitet wird,
**gekennzeichnet durch** die Merkmale:
e. die beiden inneren Gehäusebauteile (70, 72) sind zur isolierten Abdichtung der flüssigkeitsführenden Abschnitte in einer ersten Montagerichtung (4) aneinander gedrückt, und
f. die beiden inneren Gehäusebauteile (70, 72) sind in einer von der ersten Montagerichtung (4) abweichenden zweiten Montagerichtung (6) in das Außengehäuse-Bauteil (62) eingeschoben, so dass das Außengehäuse-Bauteil (62) eine Trennung der beiden inneren Gehäusebauteile (70,72) unterbindet.
wobei die Austragvorrichtung (30) vorzugsweise zusätzlich die Merkmale eines der vorstehenden Ansprüche aufweist.

13. Austragvorrichtung (30) nach Anspruch 12 mit einem dem folgenden Merkmale:
a. die Austragöffnung (65) zum Austrag der Flüssigkeit ist fluchtend zu einem Flüssigkeitsauslass (75) an einem der inneren Gehäusebauteile (72) ausgerichtet, und/oder
b. das Außengehäuse-Bauteil (62) umfasst mindestens eine Rastkante, hinter der der Verbund aus den beiden inneren Gehäusebauteilen (70, 72) einrastet, wenn diese in das Außengehäuse-Bauteil (62) eingeschoben werden und/oder
c. das Außengehäuse-Bauteil (62) weist mindestens eine Montageschräge (62B) auf, mittels derer beim Einführen der beiden inneren Gehäusebauteile (70, 72) in das Außengehäuse-Bauteil (62) die inneren Gehäusebauteile (70, 72) aneinandergedrückt werden und/oder
d. die beiden inneren Gehäusebauteile (70, 72) definieren einen Innenraum, in den eine Düseneinheit (78) und/oder eine Filtereinheit (76) eingesetzt sind.

14. Flüssigkeitsspender (10) zum Austrag kosmetischer oder pharmazeutischer Flüssigkeiten, insbesondere in Form eines Inhalator, mit den folgenden Merkmalen:
a. der Flüssigkeitsspender (10) verfügt über einen Flüssigkeitsspeicher (12) und
b. der Flüssigkeitsspender (10) verfügt über eine Austragvorrichtung (30) zum Austrag von Flüssigkeit aus dem Flüssigkeitsspeicher (12),
**gekennzeichnet durch** die Merkmale:
c. die Austragvorrichtung (30) ist nach einem der vorstehenden Ansprüche ausgebildet.

15. Flüssigkeitsspender (10) nach Anspruch 14 mit mindestens einem der folgenden Merkmale:
a. der Flüssigkeitsspeicher (12) ist als Druckspeicher ausgebildet, und/oder
b. der Flüssigkeitsspeicher (12) ist zur Aufnahme von maximal 500 ml ausgelegt, vorzugsweise von maximal 250 ml und/oder
c. der Flüssigkeitsspeicher (12) ist befüllt mit:
- einer salzhaltigen wässrigen Lösung oder
- einer wässrigen Lösung in Form einer Ringerlösung oder einer gepufferten Lösung oder
- einer wässrigen Lösung mit mindestens einem der Zusätze Kohlenhydrate, ätherische Öle, Menthol und Pflanzenextrakte oder
- einer wässrigen Lösung, enthaltend Vitamine, Spurenelemente, Mangan oder Zink, oder
- einer wässrigen Lösung mit mindestens einem der Zusätze aus der Gruppe umfassend Zimtöl, Teebaumöl, Salbeiöl, Thymianöl, Zitronenmelissenöl und/oder
d. der Flüssigkeitsspender (10) ist als Inhalator ausgebildet und verfügt über ein Atemstück (64), welches als Mundstück oder als Atemmaske ausgestaltet ist, und/oder
e. das Atemstück (64) weist einen Kanalabschnitt auf, der in einer von einer Betätigungsrichtung des Austragskopfs abweichenden Richtung ausgerichtet ist und vorzugsweise mit dieser einen Winkel zwischen 45° und 135° einschließt, insbesondere vorzugsweise einen Winkel zwischen 70° und 110°.
